# FASCICULE DE BREVET EUROPEEN

(11) **EP 3 215 835 B1**
(45) Date de publication et mention de la délivrance du brevet: **28.08.2019**
(21) Numéro de dépôt: 15808706.4
(22) Date de dépôt: 04.11.2015
(51) Int. Cl.: G01N 27/22, G01N 33/22, G01F 1/64

(54) **ENSEMBLE DE MESURE CAPACITIVE DU TAUX DE GAZ DANS UN ECOULEMENT FLUIDE**
EINHEIT ZUR KAPAZITIVEN MESSUNG DES GASGEHALTS IN EINER FLIESSENDEN FLUSSIGKEIT
CAPACITIVE MEASURING UNIT OF THE GAS CONCENTRATION IN A FLOWING FLUID

(30) Priorité: 07.11.2014 FR 1460787
(43) Date de publication de la demande: 13.09.2017
(73) Titulaire: SAFRAN AIRCRAFT ENGINES, 75015 Paris (FR); Capaab, 92290 Chatenay Malabry (FR)
(72) Inventeur: BRUERE, Alain, 92290 Chatenay Malabry (FR); NIVET, Philippe, 27420 Cantiers (FR); FAMMERY, Simon, 27190 La Bonneville sur Iton (FR)
(74) Mandataire: Gilbey, Vincent
(86) Numéro de dépôt international: PCT/FR2015/052981
(87) Numéro de publication internationale: WO 2016/071635

(56) Documents cités:
- EP-A2- 0 488 507
- WO-A1-94/03802
- US-A- 3 374 672
- US-A- 4 751 842
- US-A1- 2007 186 679
- US-B1- 6 467 358

## Description

### DOMAINE TECHNIQUE GENERAL

La présente invention concerne le domaine des systèmes de mesure de teneur en gaz dans une conduite d'alimentation.

### ETAT DE L'ART

La connaissance de la teneur en gaz dans une conduite d'alimentation d'un moteur est un paramètre important pour le recalage du modèle de fonctionnement appliqué au moteur en fonction du temps, en particulier pour les moteurs d'engins spatiaux ou d'aéronefs, qui sont typiquement recalés par rapport à des données expérimentales.

Des capteurs ont déjà été proposés pour réaliser de telles mesures. On peut notamment citer le document WO 2013017795 qui détaille un tel capteur. Le capteur présenté dans ce document présente toutefois plusieurs inconvénients.
En premier lieu, il s'agit d'un capteur ayant des dimensions importantes avec un diamètre de l'ordre de 120 mm, qui n'est pas adapté à des conduites de faibles dimensions. De plus, le capteur présenté délivre un signal de sortie multiplexé, ce qui est pénalisant pour l'analyse des résultats. Enfin, le capteur comprend des électrodes et des tiges de fixation positionnées au sein de l'écoulement, ce qui entraine des pertes de charges et est donc fortement pénalisant.

Le document EP 0488507 présente également un capteur pour la détermination du pourcentage d'eau dans un écoulement de fluide.
Ce document présente plusieurs configurations d'électrodes, de sorte que le champ généré soit parallèle ou perpendiculaire à l'écoulement de fluide dans une conduite. Ce document propose notamment d'intégrer plusieurs électrodes de mesure dans les parois d'une conduite d'écoulement de fluide, ces électrodes étant chacune isolées électriquement du corps de la conduite. Le système proposé dans ce document détermine le pourcentage d'eau dans l'écoulement au moyen de la tension et de l'intensité appliquées aux électrodes, de la tension mesurée entre les électrodes, et de la température du fluide au point de mesure.

Le document US 6467358 présente quant à lui une méthode de mesure du taux de différents fluides dans un écoulement multiphasique, au moyen d'un système comprenant trois électrodes disposées dans une canalisation, et chacune isolée électriquement de la canalisation. Cette méthode repose sur l'utilisation d'un système de mesure de capacité électrostatique ou d'impédance au moyen de mesures effectuées via les trois électrodes.

La présente invention vise ainsi à proposer une solution améliorant au moins partiellement ces aspects.

### PRESENTATION DE L'INVENTION

A cet effet, la présente invention propose un ensemble comprenant une conduite amont, une conduite aval, et un système de mesure de la variation de teneur en gaz d'un écoulement diphasique, ledit système comprenant
une gaine isolante présentant une extrémité amont et une extrémité aval,
une masse amont, une électrode de mesure, une électrode de garde et une masse aval disposées successivement dans ladite gaine isolante, et présentant chacune une section interne identique définissant un conduit interne d'écoulement d'un diphasique de la masse amont vers la masse aval, l'électrode de garde étant configurée de manière à être soumise à un même potentiel que l'électrode de mesure, l'électrode de mesure étant configurée de manière à mesurer, par rapport à la masse amont, la capacité et la variation de capacité du milieu constituant l'écoulement diphasique, l'ensemble étant caractérisé en ce que
lesdites conduites amont et aval présentent une section interne identique à la section interne dudit système de mesure,
lesdites conduites amont et aval sont connectées fluidiquement via le système de mesure, de sorte que le conduit interne du système de mesure soit dans le prolongement des conduits internes des conduites amont et aval,
la masse amont et la masse aval sont reliées électriquement à la conduite amont et à la conduite aval respectivement,
ledit ensemble comprenant en outre une unité de traitement configurée pour traiter les mesures de capacité réalisées entre l'électrode de mesure et la masse amont.

On présente ci-après différents modes de réalisation de cet ensemble, pouvant être pris isolément ou en combinaison.

L'ensemble peut comprendre en outre une entretoise isolante disposée dans la gaine isolante, entre l'électrode de mesure et la masse amont, présentant une section interne identique à celle de l'électrode de mesure et de la masse amont, de manière à isoler électriquement la masse amont par rapport à l'électrode de mesure.
L'électrode de garde peut comprendre un fourreau de garde comprenant une portion cylindrique s'étendant jusqu'à la masse amont, entre la gaine isolante d'une part, et l'électrode de mesure, l'entretoise isolante et la masse amont d'autre part.
La masse aval peut alors comprendre un fourreau interne comprenant une portion cylindrique s'étendant le long de la gaine isolante, entre la gaine isolante d'une part, et l'électrode de masse et la masse amont d'autre part.

### PRESENTATION DES FIGURES

D'autres caractéristiques, buts et avantages de l'invention ressortiront de la description qui suit, qui est purement illustrative et non limitative, et qui doit être lue en regard des figures annexées, sur lesquelles :
- la figure 1 présente une vue partielle d'un système selon un aspect de l'invention ;
- la figure 2 présente une vue en coupe d'un tel système,
- la figure 3 représente schématiquement les lignes de champ au sein d'un tel système.

Sur l'ensemble des figures, les éléments en commun sont repérés par des références numériques identiques.

### DESCRIPTION DETAILLEE

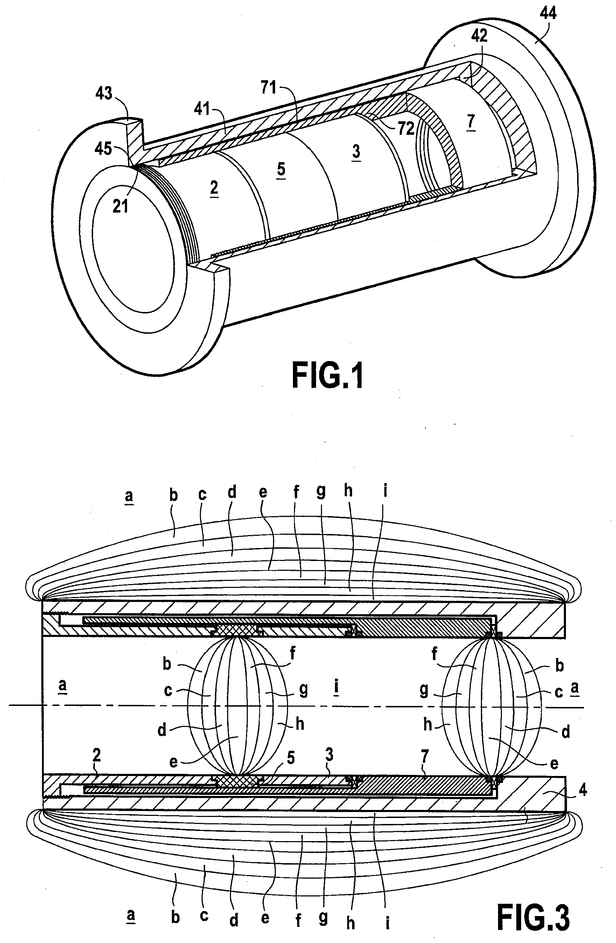

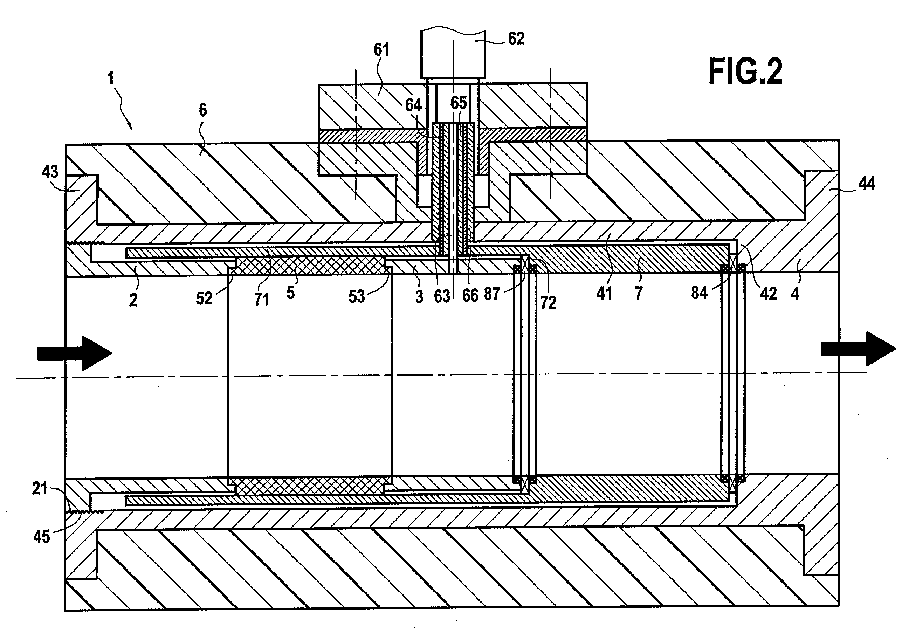

Les figures 1 et 2 présentent deux vues d'un système selon un aspect de l'invention.

On représente sur ces figures un système 1 de mesure de la variation de la teneur en gaz dans un écoulement diphasique.

Le système 1 tel qu'illustré comprend une masse amont 2, une électrode de mesure 3 et une masse aval 4 disposées successivement dans une gaine isolante 6 (non représentée sur la figure 1), réalisant une isolation thermique et électrique.
L'électrode de mesure 3, la masse amont 2 et la masse aval 4 forment un système d'électrodes ; l'électrode de mesure 3 forme typiquement la cathode, tandis que la masse amont 2 et la masse aval 4 forment alors des anodes.

L'électrode de mesure 3 est disposée de manière à être séparée de la masse amont 2, de manière à ce que l'électrode de mesure 3 et la masse amont 2 ne soient pas en contact électrique direct.
Le système comprend ainsi par exemple une entretoise isolante 5, définissant un espacement minimum entre l'électrode de mesure 3 et la masse amont 2.

La masse amont 2, l'entretoise isolante 5, l'électrode de mesure 3 et la masse aval 4 ont chacune une section interne identique, de manière à définir un conduit interne de section constante lorsqu'ils sont mis bout à bout, par exemple de section tubulaire, cylindrique de révolution.

La masse amont 2 et la masse aval 4 sont réalisées en matériau conducteur, et forment la structure d'une portion d'une conduite d'écoulement d'un fluide diphasique.
Lors de l'utilisation du système, la masse amont 2 et la masse aval 4 sont ainsi reliées directement à la masse électrique de l'installation, au même titre que la conduite dans lequel le système est inséré.

L'entretoise isolante 5 est disposée entre la masse amont 2 et l'électrode de mesure 3 ; l'entretoise isolante 5 est réalisée en un matériau isolant électriquement de manière à les isoler électriquement l'une par rapport à l'autre.
L'électrode de mesure 3 permet donc de réaliser une mesure de la permittivité d'un diphasique circulant dans le conduit interne ainsi formé par le système 1, ledit diphasique circulant de la masse amont 2 vers la masse aval 4, comme indiqué par les flèches sur la figure 2.

Une telle mesure de la variation de la permittivité permet de détecter les variations de taux de gaz au sein du diphasique.
En effet, la permittivité d'un milieu liquide varie proportionnellement en fonction du taux de gaz dans le liquide. Ainsi, en chargeant électriquement la masse amont 2, on peut relever le potentiel de l'électrode de mesure, l'évolution de la différence de capacité mesurée traduisant alors directement l'évolution du taux de gaz au sein du diphasique circulant dans le conduit interne.

Les mesures ainsi réalisées sont par exemple traitées au moyen d'une unité de conditionnement chargée de convertir la variation de capacité en une variation d'une grandeur électrique (tension, courant, fréquence...), laquelle peut notamment être reliée à un ordinateur ou toute autre unité de traitement adaptée.

De plus, en reliant la masse amont 2 et la masse aval 4 à la masse électrique de l'installation, la sensibilité du système 1 proposé aux interférences est réduite, le traitement des mesures est ainsi simplifié par rapport aux systèmes de mesure conventionnels dans lesquels les éléments formant l'anode et la cathode du système 1 sont isolés électriquement de la conduite. Le système proposé utilise en effet la masse électrique de l'installation comme référence du potentiel électrique, contrairement aux systèmes antérieurs pour lesquels les différentes électrodes sont chacune isolées de la conduite et donc de la masse de l'installation.

Le système proposé permet donc de réaliser un capteur capacitif, dont au moins une des électrodes (ici les masses amont 2 et aval 4, formant typiquement des anodes) est formée par la conduite elle-même, et est donc reliée à la masse électrique de l'installation de la conduite.

Le système 1 est adapté pour être intégré dans une ligne de circulation de diphasique, par exemple une ligne d'alimentation en ergol d'un engin spatial. Le système 1 est alors interposé entre une conduite amont et une conduite aval auxquelles il est relié par exemple au moyen de brides ou de connecteurs.

Le conduit interne défini par le système 1 a alors une section identique à la section des conduites amont et aval auxquelles il est destiné à être connecté.

Le système 1 permet donc de mesurer l'évolution du taux de gaz au sein d'un écoulement diphasique, sans générer des pertes de charge dans cet écoulement.

Les différents composants du système 1 sont en effet configurés de manière à être dans le prolongement de la conduite de l'écoulement, et non pas au sein de l'écoulement comme on l'observe dans les systèmes connus.

De plus, dans le système 1 proposé, les composants réalisant les mesures permettant de mesurer l'évolution du taux de gaz au sein d'un écoulement diphasique ne sont pas limités en termes de dimensions. Le système 1 peut donc être adapté sur des canalisations de toutes dimensions, par exemple des canalisations ayant un diamètre compris entre 4 et 60 millimètres.

Par ailleurs, un tel système 1 permet d'acquérir des données de manière directe, sans multiplexage, ce qui facilite considérablement l'exploitation des mesures par rapport aux systèmes antérieurs qui génèrent des données multiplexées.

En outre, dans le cas où les différentes électrodes présentent une section interne cylindrique de révolution, la réponse mesurée est alors peu dépendante de la position radiale des bulles d'air dans l'écoulement diphasique.

Le système 1 comprend également une électrode de garde 7, soumise au même potentiel que l'électrode de mesure 3, afin de compenser les perturbations électriques provoquées notamment par la masse aval 4 et par d'autres éléments mécaniques du système 1 reliés à la masse amont 2 ou aval 4. L'électrode de garde 7 est alors interposée entre l'électrode de mesure 3 et la masse aval 4, sans être au contact ni de l'électrode de mesure 3, ni de la masse aval 4.

Dans le mode de réalisation représenté sur les figures, l'électrode de garde 7 comprend un fourreau de garde 71 présentant une portion cylindrique s'étendant jusqu'à la masse amont 2, entre la gaine isolante 1 d'une part, et l'électrode de mesure 3, l'entretoise isolante 5 et la masse amont 2 d'autre part, sans être au contact ni de l'électrode de mesure 3, ni de la masse amont 2. L'entretoise isolante 5 peut ainsi présenter un diamètre externe sensiblement supérieur à celui de l'électrode de mesure 3 et de la masse amont 2, de manière à assurer un espacement entre l'électrode de garde 7 et l'électrode de mesure 3 et la masse amont 2.

La masse aval 4 présente quant à elle un fourreau interne 41, s'étendant entre la gaine isolante 6 d'une part, et l'électrode de mesure 3, l'entretoise isolante 5 la masse amont 2 et le cas échant l'électrode de garde 7 d'autre part.

Comme on le voit sur les figures, le fourreau de garde 71 isole ainsi l'électrode de mesure 3 et l'entretoise 5 de la masse aval 4, le fourreau de garde 71 étant interposé entre l'électrode de mesure 3 et l'entretoise 5 d'une part, et le fourreau interne 41 de la masse aval 4 d'autre part.

Dans le mode de réalisation représenté sur les figures, la masse aval 4 forme une extrémité aval du système 1. La masse aval 4 comprend un épaulement 42 à partir duquel s'étend le fourreau interne 41, et formant une butée contre laquelle est positionnée l'électrode de garde 7.
De la même manière, l'électrode de garde 7 comprend un épaulement 72 à partir duquel s'étend le fourreau de garde 71, l'épaulement 72 formant une butée contre laquelle est positionnée l'électrode de mesure 3.

Dans le mode de réalisation représenté sur la figure 2, on positionne donc dans un premier temps la masse aval 4 dans la gaine isolante 6. On insère ensuite l'électrode de garde 7 via l'extrémité amont, de manière à ce qu'elle vienne en butée contre l'épaulement de la masse aval 4. Un élément d'étanchéité 84 est typiquement disposé entre l'électrode de garde 7 et la masse aval 4, de manière à isoler électriquement l'électrode de garde 7 de la masse aval 4.
L'électrode de mesure 3 est ensuite insérée via l'extrémité amont, jusqu'à venir en butée contre l'épaulement 72 de l'électrode de garde 71. Un élément d'étanchéité 87 est typiquement disposé entre l'électrode de garde 7 et la masse aval 4.
L'entretoise 5 est ensuite insérée via l'extrémité amont, jusqu'à venir en butée contre l'électrode de mesure 3. Enfin, la masse amont 2 est insérée via l'extrémité amont, jusqu'à venir en butée contre l'entretoise 5. L'entretoise 5 comprend typiquement des moyens de centrage 52 et 53, de manière à centrer l'entretoise 5 respectivement par rapport à la masse amont 2 et à l'électrode de mesure 3.

La masse amont 2 comprend par exemple une portion filetée 21, adaptée pour être vissée sur une portion filetée 45 du fourreau interne 41 de la masse aval 4, formant par exemple l'extrémité amont du système 1.
En vissant ainsi la masse amont 2 par rapport à la masse aval 4, la masse amont 2 réalise un maintien des différents éléments du système 1 disposés entre la masse amont 2 et la masse aval 4, à savoir l'entretoise isolante 5, l'électrode de mesure 3 et l'électrode de garde 7 dans le mode de réalisation représenté.

Dans le mode de réalisation représenté, la masse aval 4 présente deux collerettes radiales à ses deux extrémités, respectivement une collerette amont 43 et une collerette aval 44.

La gaine isolante 6 est typiquement munie d'un support de bride 61 permettant de connecter le système 1 par exemple à un contrôleur, et comprenant par exemple une prise coaxiale 62 étanche et isolée, reliée aux électrodes de mesure 3 et de garde 7.
La prise coaxiale 62 telle que représentée sur la figure 2 comprend :
- une âme 63, reliée à l'électrode de mesure 3,
- une garde 64, disposée autour de l'âme 63, connectée à l'électrode de garde 7,
- un isolant interne 65, disposé entre l'âme 63 et la garde 64, et
- un isolant externe 66, disposé autour de la garde 64.

En fonctionnement, les potentiels appliqués aux électrodes génèrent un champ électrostatique.
Or, on comprend bien que la nature du champ électrostatique ainsi généré influe sur la sensibilité des mesures effectuées par le système 1. Le champ électrostatique généré dépendant lui-même notamment des dimensions des différents composants et de leurs positionnements relatifs, les dimensions et le positionnement des différents composants du système 1 influent donc sur la sensibilité du système 1.

On représente ainsi sur la figure 3 les différents éléments du système 1 tel que présenté précédemment, ainsi que les lignes de champ générées lors de son fonctionnement.
On désigne les différentes valeurs du champ électrostatique par des lettres de a à i. Le potentiel va en croissant de la lettre a, qui désigne le potentiel le plus faible, à la lettre i qui désigne le potentiel le plus élevé.

Comme mentionné précédemment, la mesure de la variation de la permittivité étant réalisée entre l'électrode de mesure 3 sur la portion du système 1 correspondant à l'écart entre l'électrode de mesure 3 et la masse amont 2, c'est-à-dire la portion du système correspondant à l'entretoise isolante 5 dans le cas où le système 1 comprend une telle entretoise isolante 5 entre l'électrode de mesure 3 et la masse amont 2. Par conséquent, afin d'accroître la sensibilité du système 1, on cherche à avoir une variation maximale du potentiel sur cette portion du système 1.
Ainsi, les différents composants du système sont avantageusement configurés de manière à ce qu'un maximum de lignes de champ se situent au sein de la portion du système 1 correspondant à l'écart entre l'électrode de mesure 3 et la masse amont 2, c'est-à-dire la portion du système correspondant à l'entretoise isolante 5 dans le cas où le système 1 comprend une telle entretoise isolante 5 entre l'électrode de mesure 3 et la masse amont 2.

L'électrode de mesure 3 est typiquement disposée de manière à être peu éloignée de la masse amont 2, par exemple à une distance comprise entre 10 et 20 mm dans le cas d'un conduit de 29 mm de diamètre, ou encore de l'ordre de 15mm. L'entretoise isolante 5 a par conséquent une dimension adaptée de manière à garantir cet espacement.

Selon un mode de réalisation particulier, l'électrode de mesure 3 peut être subdivisée en plusieurs secteurs, par exemple quatre secteurs correspondant chacun sensiblement à un quart de cercle dans le cas d'un écoulement dans une conduite tubulaire. Les différents secteurs peuvent par exemple être isolés les uns des autres par des portions de matériau isolant,
Les mesures effectuées sur chacun des secteurs permettent alors de détecter dans quel secteur sont situées les bulles de gaz de l'écoulement diphasique, et permettent ainsi de déterminer la proportion de gaz dans les différents secteurs de l'écoulement diphasique.

## Revendications

1. Ensemble comprenant une conduite amont, une conduite aval, et un système (1) de mesure de la variation de teneur en gaz d'un écoulement diphasique, ledit système (1) comprenant
- une gaine isolante (6) présentant une extrémité amont et une extrémité aval,
- une masse amont (2) réalisée en matériau conducteur, une électrode de mesure (3), une électrode de garde (7) et une masse aval (4) réalisée en matériau conducteur disposées successivement dans ladite gaine isolante (6), et présentant chacune une section interne identique définissant un conduit interne d'écoulement d'un diphasique de la masse amont (2) vers la masse aval (4), l'électrode de garde (7) étant configurée de manière à être soumise à un même potentiel que l'électrode de mesure (3), l'électrode de mesure (3) étant configurée de manière à mesurer, par rapport à la masse amont (2), la capacité et la variation de capacité du milieu constituant l'écoulement diphasique,
l'ensemble étant **caractérisé en ce que**
lesdites conduites amont et aval présentent une section interne identique à la section interne dudit système de mesure (1),
lesdites conduites amont et aval sont connectées fluidiquement via le système (1) de mesure, de sorte que le conduit interne du système de mesure soit dans le prolongement des conduits internes des conduites amont et aval,
la masse amont (2) et la masse aval (4) sont reliées électriquement à la conduite amont et à la conduite aval respectivement,
ledit ensemble comprenant en outre une unité de traitement configurée pour traiter les mesures de capacité réalisées entre l'électrode de mesure (3) et la masse amont (2).

2. Ensemble (1) selon la revendication 1, comprenant en outre une entretoise isolante (5) disposée dans la gaine isolante (6), entre l'électrode de mesure (3) et la masse amont (2), présentant une section interne identique à celle de l'électrode de mesure (3) et de la masse amont (2), de manière à isoler électriquement la masse amont (2) par rapport à l'électrode de mesure (3).

3. Ensemble (1) selon la revendication 2, dans lequel ladite électrode de garde (7) comprend un fourreau de garde (71) comprenant une portion cylindrique s'étendant jusqu'à la masse amont (2), entre la gaine isolante (6) d'une part, et l'électrode de mesure (3), l'entretoise isolante (5) et la masse amont (2) d'autre part.

4. Ensemble (1) selon la revendication 3, dans lequel la masse aval (4) comprend un fourreau interne(41) comprenant une portion cylindrique s'étendant le long de la gaine isolante (6), entre la gaine isolante (6) d'une part, et l'électrode de mesure (3) et la masse amont (2) d'autre part.

## Patentansprüche

1. Baugruppe, die eine vorgelagerte Leitung, eine nachgelagerte Leitung und ein System (1) zur Messung der Veränderung des Gasgehalts einer Zweiphasenströmung umfasst, wobei das System (1) umfasst
- eine isolierende Hülle (6), die ein vorgelagertes Ende und ein nachgelagertes Ende aufweist,
- eine vorgelagerte Masse (2), die mit einem leitfähigen Material umgesetzt wird, eine Messelektrode (3), eine Schutzelektrode (7) und eine nachgelagerte Masse (4), die mit einem leitfähigen Material umgesetzt wird, die nacheinander in der isolierenden Hülle (6) angeordnet sind und jeweils einen identischen inneren Abschnitt aufweisen, der eine innere Leitung einer Zweiphasenströmung von der vorgelagerten Masse (2) zu der nachgelagerten Masse (4) definiert, wobei die Schutzelektrode (7) dazu ausgestaltet ist, einem gleichen Potenzial zu unterliegen wie die Messelektrode (3), wobei die Messelektrode (3) dazu ausgestaltet ist, in Bezug auf die vorgelagerten Masse (2) die Kapazität und die Veränderung der Kapazität des Mediums zu messen, das die Zweiphasenströmung bildet,
wobei die Baugruppe **dadurch gekennzeichnet ist, dass**
die vorgelagerte und nachgelagerte Leitung einen inneren Abschnitt aufweisen, der mit dem inneren Abschnitt des Messsystems (1) identisch ist,
die vorgelagerte und nachgelagert Leitung fluidisch über das Messsystem (1) verbunden sind, sodass sich die innere Leitung des Messsystems in der Verlängerung der inneren Leitungen der vorgelagerten und nachgelagerten Leitung befindet,
die vorgelagerte Masse (2) und die nachgelagerte Masse (4) jeweils an der vorgelagerten Leitung und an der nachgelagerten Leitung elektrisch verbunden sind und
die Baugruppe ferner eine Behandlungseinheit umfasst, die dazu ausgestaltet ist, die Messungen der Kapazität zu behandeln, die zwischen der Messelektrode (3) und der vorgelagerten Masse (2) ausgeführt werden.

2. Baugruppe (1) nach Anspruch 1, die ferner einen isolierenden Abstandshalter (5), der in der isolierenden Hülle (6) zwischen der Messelektrode (3) und der vorgelagerten Masse (2) angeordnet ist, umfasst und einen inneren Abschnitt aufweist, der mit dem der Messelektrode (3) und der vorgelagerten Masse (2) identisch ist, um die vorgelagerte Masse (2) in Bezug auf die Messelektrode (3) elektrisch zu isolieren.

3. Baugruppe (1) nach Anspruch 2, wobei die Schutzelektrode (7) eine Schutzummantelung (71) umfasst, die einen zylindrischen Teil umfasst, der sich bis zu der vorgelagerten Masse (2), zwischen der isolierenden Hülle (6) einerseits, und der Messelektrode (3), dem isolierenden Abstandshalter (5) und der vorgelagerten Masse (2) andererseits, erstreckt.

4. Baugruppe (1) nach Anspruch 3, wobei die nachgelagerte Masse (4) eine innere Ummantelung (41) umfasst, die einen zylindrischen Teil umfasst, der sich entlang der isolierenden Hülle (6), zwischen der isolierenden Hülle (6) einerseits, und der Masselektrode (3) und der vorgelagerten Masse (2) andererseits, erstreckt.

## Claims

1. An assembly comprising an upstream pipe, a downstream pipe, and a system (1) for measuring variation in gas content within a two-phase flow, said system (1) comprising:
• an insulating sheath (6) presenting an upstream end and a downstream end; and
• an upstream ground (2) made in conductive material, a measurement electrode (3), a guard electrode (7), and a downstream ground (4) made in conductive material arranged in succession in said insulating sheath (6) and each presenting an identical internal section defining an internal flow duct for a two-phase flow from the upstream ground (2) towards the downstream ground (4), the guard electrode (7) being configured so as to be subjected to the same potential as the measurement electrode (3), the measurement electrode (3) being configured so as to measure the capacitance of the medium constituting the two-phase flow relative to the upstream ground (2), and to measure variation of that capacitance;
the assembly being **characterized in that**:
• said upstream and downstream pipes present an internal section identical to the internal section of said measurement system (1);
• said upstream and downstream pipes are in fluid flow connection with each other via the measurement system (1) so that the internal duct of the measurement system extends the internal ducts of the upstream and downstream pipes; and
• the upstream ground (2) and the downstream ground (4) are electrically connected to the upstream pipe and to the downstream pipe, respectively;
said assembly further comprising a processor unit configured to process the capacitance measurements taken between the measurement electrode (3) and the upstream ground (2).

2. An assembly (1) according to claim 1, further comprising an insulating spacer (5) arranged in the insulating sheath (6) between the measurement electrode (3) and the upstream ground (2), the spacer presenting an internal section identical to that of the measurement electrode (3) and of the upstream ground (2) so as to insulate the upstream ground (2) electrically from the measurement electrode (3).

3. An assembly (1) according to claim 2, wherein said guard electrode (7) comprises a guard sheath (71) having a cylindrical portion extending to the upstream ground (2) between the insulating sheath (6) on one side and the measurement electrode (3), the insulating spacer (5), and the upstream ground (2) on the other side.

4. An assembly (1) according to claim 3, wherein the downstream ground (4) comprises an internal sheath (41) comprising a cylindrical portion extending along the insulating sheath (6) between the insulating sheath (6) on one side and the measurement electrode (3) and the upstream ground (2) on the other side.
